# FASCICULE DE BREVET EUROPEEN

(11) **EP 0 654 249 B1**
(45) Date de publication et mention de la délivrance du brevet: **10.03.1999**
(21) Numéro de dépôt: 94420322.3
(22) Date de dépôt: 17.11.1994
(51) Int. Cl.: A61B 17/60

(54) **Prothese intervertébrale extradiscale**
Intervertebralen, ausserhalb der Bandscheibe angeordnete Prothese
Extradiscal intervertebral prosthesis

(30) Priorité: 17.05.1994 FR 9406227; 18.11.1993 FR 9314024
(43) Date de publication de la demande: 24.05.1995
(73) Titulaire: Graf, Henry, F-69006 Lyon (FR)
(72) Inventeur: Graf, Henry, F-69006 Lyon (FR)
(74) Mandataire: Obolensky, Michel

(56) Documents cités:
- EP-A- 0 322 334
- EP-A- 0 498 709
- EP-A- 0 516 567
- FR-A- 2 662 073
- US-A- 4 743 260

## Description

La présente invention a trait aux prothèses intervertébrales extradiscales agissant en dehors du disque en vue de compenser des défauts du rachis.

On connaît des systèmes comportant un ligament en forme d'anneau associé à des vis pédiculaires engagées dans au moins deux vertèbres voisines présentant une pathologie de leur disque intervertébral. De tels systèmes présentent l'inconvénient de se rompre parfois sous l'action des efforts répétés d'extension qu'ils supportent, du fait qu'ils sont réalisés en textile. En outre, ils provoquent des fibroses, comme tous les textiles implantés dans le corps humain. Ce genre de système contrôle imparfaitement la variation en flexion-extension intervertébrale et ne permet pas une approche précise du repositionnement vertébral et du contrôle du mouvement résiduel entre les deux vertèbres.

On connaît, par EP-A1-0 516 567, un dispositif de stabilisation intervertébrale comprenant un amortisseur de forme allongée pourvu d'une partie centrale ventrue reliée par deux cols à deux extrémités renflées. Chacune de ces extrémités est assujettie à un implant fixé sur une vertèbre correspondante. Ce dispositif est donc apte à résister d'une part à un allongement et d'autre part à une compression.

La présente invention vise à remédier à ces inconvénients et à permettre la réalisation d'une prothèse intervertébrale extradiscale mieux supportée par l'organisme, qui ne présente pas de risque de rupture et contrôle précisément le mouvement résiduel dans le sens de la flexion et/ou extension vertébrale. Ce contrôle est celui de la variation de la distance d'un pédicule à l'autre de deux vertèbres voisines lors du mouvement de flexion et/ou extension.

A cet effet, l'invention a pour objet une prothèse intervertébrale extradiscale munie de moyens d'accrochage directs ou indirects à deux vertèbres du rachis d'un patient, comprenant un corps allongé totalement ou partiellement fermé comportant une chambre de compression, et une rotule associée à un premier moyen de fixation engagé dans une première vertèbre, ledit corps étant accroché à une autre vertèbre (8) ou étant relié à un autre moyen de fixation implanté dans une autre vertèbre, directement ou indirectement par l'intermédiaire d'au moins un moyen de liaison qui est en appui contre ledit autre moyen de fixation, caractérisée en ce que ladite rotule est retenue à l'intérieur de la chambre de compression, une unique extrémité de ladite chambre étant solidaire d'un bloc élastique dont une face est en appui contre la rotule.

Dans une forme de réalisation préférée, la face libre du bloc élastique affecte la forme d'une portion concave de sphère de même diamètre que la rotule précitée. Pour protéger cette face libre, on peut lui adjoindre une coquille rigide ayant la forme d'une calotte sphérique concave.

Suivant une première forme d'exécution, l'extrémité du corps opposée à celle qui porte le bloc élastique est en appui contre la tige d'un crochet solidaire d'un moyen de fixation implanté dans l'autre vertèbre.

Il est également possible de relier au crochet l'extrémité en question du corps, c'est-à-dire celle qui est opposée au bloc élastique, au moyen d'un maillon.

Enfin, l'invention concerne encore la combinaison d'une prothèse telle que décrite ci-dessus avec une cale élastique engagée entre les deux vertèbres recevant les vis pédiculaires, ladite cale remplaçant par exemple une partie lésée du disque intervertébral correspondant.

Le dessin annexé, donné à titre d'exemple, permettra de mieux comprendre l'invention, les caractéristiques qu'elle présente et les avantages qu'elle est susceptible de procurer :
Fig. 1 et 1A sont des vues montrant les différents éléments d'une prothèse intervertébrale extradiscale suivant l'invention.
Fig. 2 à 2C sont des vues semblables à celles de fig. 1, mais illustrant une première variante de la prothèse intervertébrale extradiscale.
Fig. 3 est une vue semblable à celle de fig. 2, mais illustrant une seconde variante de la prothèse intervertébrale suivant l'invention.
Fig. 4 à 6 sont des vues en perspective montrant plusieurs variantes de la prothèse intervertébrale extradiscale suivant l'invention.
Fig. 7 est une vue semblable à celle de fig. 4, mais illustrant une prothèse intervertébrale dont la tige de liaison peut coulisser par rapport au corps de ladite prothèse.
Fig. 8 et 9 sont des coupes illustrant une autre variante de la prothèse intervertébrale.
Fig. 10 est une vue représentant l'association de la prothèse intervertébrale suivant l'invention avec une tige ou plaque d'un fixateur d'ostéosynthèse.
Fig. 11 est une vue montrant une variante des moyens de fixation dans les vertèbres d'une colonne vertébrale de la prothèse inter-vertébrale.
Fig. 12 est une vue illustrant une variante d'accrochage des tiges ou plaques sur la vertèbre voisine à réunir.
Fig. 13 et 14 sont des vues représentant une variante de la prothèse intervertébrale.
Fig. 15 à 17 sont des vues montrant une dernière variante de prothèse intervertébrale extradiscale.

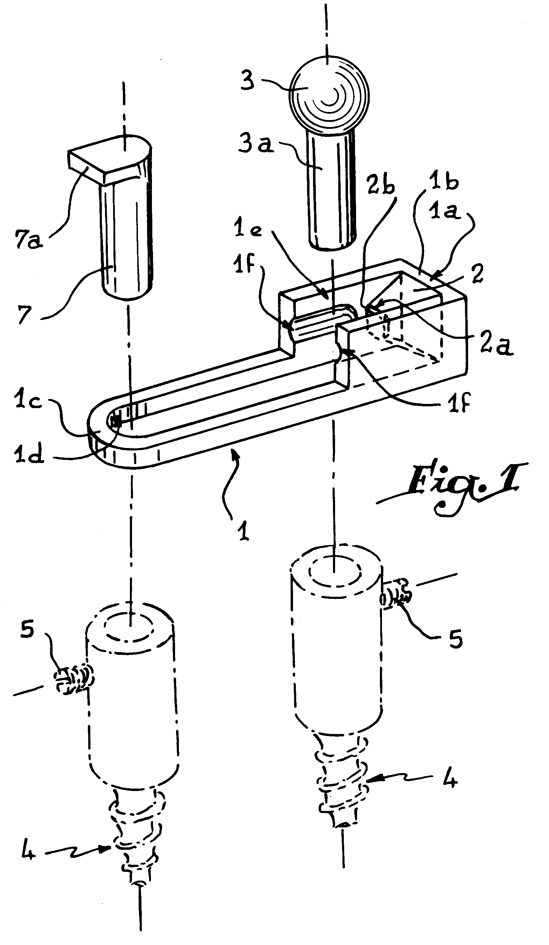

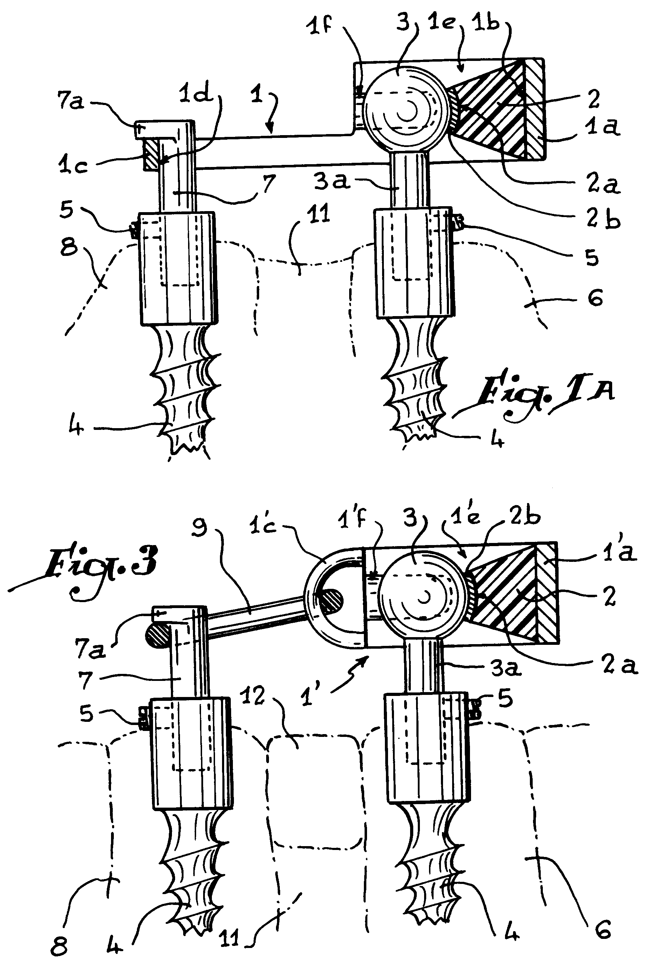

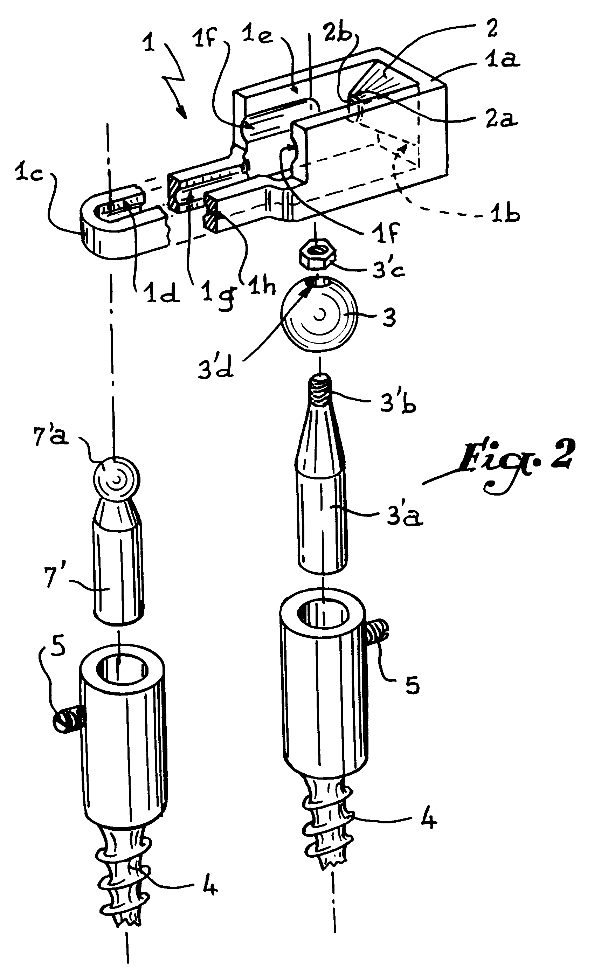

La prothèse suivant l'invention illustrée en fig. 1 et 1A comprend essentiellement un corps rigide 1, affectant une forme allongée et ouverte dont l'extrémité 1a est épaissie de manière à présenter une hauteur beaucoup plus importante que le reste du corps pour délimiter une chambre de compression 1e. L'extrémité épaissie 1a de la chambre 1e forme un fond extrême 1b de préférence plat, mais qui pourrait être aussi arrondi. Contre le fond 1b on a assemblé par tout moyen approprié un bloc élastique 2 réalisé par exemple en un élastomère naturel ou synthétique. De préférence, le bloc est collé contre le fond 1b de l'extrémité 1a du corps 1. Le bloc 2 est avantageusement réalisé sous la forme d'un tronc de pyramide, dont la face libre 2a présente la forme d'une portion concave de sphère.

Cette face 2a est destinée à coopérer étroitement avec une rotule 3 présentant une queue 3a assemblée à un moyen de fixation ou une vis pédiculaire 4, par exemple par une vis-pression 5, comme cela est bien connu dans la pratique. Le moyen de fixation dans les vertèbres peut être également réalisé au moyen d'un crochet qui vient se glisser sous leslames de chaque vertèbre à réunir, comme on le verra mieux plus loin. La vis 4 est bien entendu implantée dans le pédicule d'une des vertèbres 6 d'un patient. La face 2a peut être revêtue, comme illustré en fig. 1A, d'une coquille rigide 2b prévue concave et de diamètre égal à celui de la rotule 3. Une telle coquille peut être réalisée en toute matière appropriée et plus particulièrement en acier inoxydable.

Les parois de la chambre de compression 1e s'étendant perpendiculairement au fond 1b comportent respectivement une empreinte débouchante 1f. Cette dernière présente une forme en portion de cercle dont le rayon est équivalent à celui de la rotule 3 pour retenir celle-ci dans ses déplacements verticaux par rapport au corps 1.

La seconde extrémité 1c du corps 1, qui présente la forme d'un U, est arrondie de manière que sa face interne 1d puisse venir en appui étroit contre une tige cylindrique 7 associée à une autre vis pédiculaire 4 implantée dans la vertèbre 8 voisine de celle 6. De manière avantageuse, la tige 7 présente une tête 7a s'étendant au-dessus de l'extrémité 1c du corps 1, comme parfaitement illustré en fig. 1A, de telle sorte qu'en réalité cette extrémité 1c coopère avec un crochet empêchant le corps 1 de basculer vers l'extérieur par rapport à la vertèbre 8.

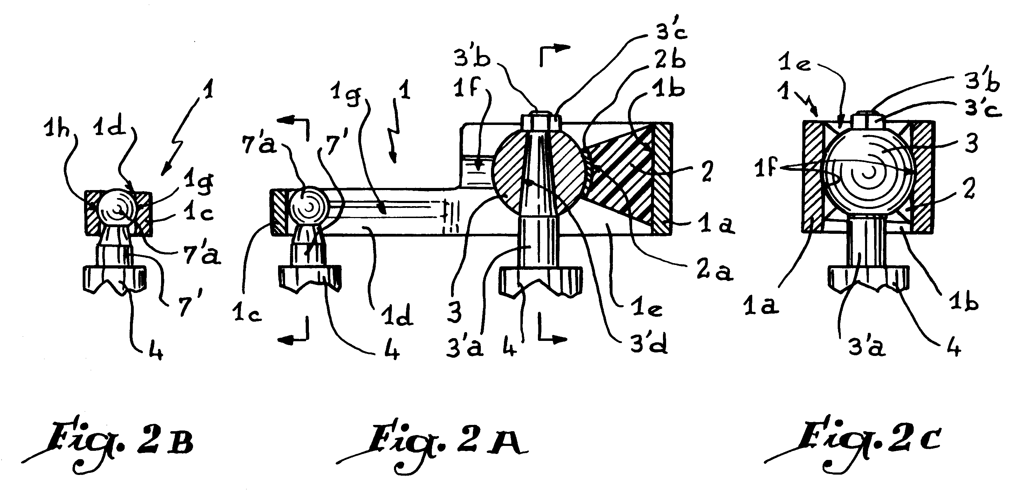

On a représenté en fig. 2 à 2C une variante de la prothèse intervertébrale extradiscale montrée en fig. 1 et 1A. En effet l'extrémité 1c comporte sur sa face interne 1d deux empreintes opposées 1g et 1h qui coopèrent avec une rotule de faible diamètre 7'a solidaire d'une tige 7' . Cette dernière est fixée sur la vis pédiculaire 4 de la même manière que pour la tige 7. Les empreintes 1g et 1h présentent un profil en portion de cercle dont le rayon est identique à celui de la rotule 7'a pour que cette dernière soit libre dans ses déplacements horizontaux et retenue dans ses mouvement verticaux.

On a montré également en fig. 2 à 2C une variante de la rotule 3 qui peut être rapportée sur une queue 3'a à profil conique se terminant par une partie filetée 3'b qui coopère avec un écrou 3'c. La rotule 3 est percée d'un trou débouchant 3'd à profil conique qui coopère avec celui de la queue 3'a, tandis que l'écrou 3'c vient immobiliser ladite rotule 3.

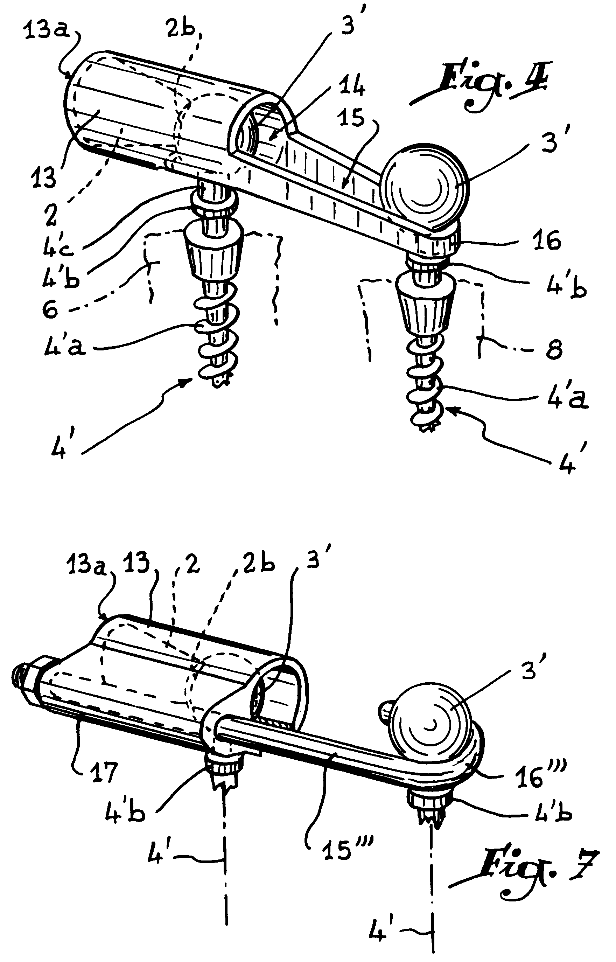

Il va de soi qu'on pourrait, comme illustré en fig. 3, changer la forme du corps référencé alors 1' de manière que sa tête 1'a identique à celle 1a du corps 1 soit associée à une simple anse 1'c correspondant à l'extrémité 1c du corps 1. Le corps 1' comprend également une chambre de compression 1'e pourvue d'une enpreinte 1'f dans laquelle est fixé le bloc élastique 2. Dans ces conditions, la liaison entre la rotule 3 et la tige 7, c'està-dire entre les vertèbres 6 et 8 est complétée par un maillon 9 entourant la tige 7 et l'anse 1'c, l'assemblage de la rotule 3 et du corps 1' restant identique à celui prévu pour le corps 1.

En fig. 4 à 6, on a représenté des variantes préférées de l'invention qui consistent à prévoir un corps cylindrique 13 dont l'une des extrémités est fermée par un fond 13a délimitant une chambre de compression 14, tandis que celle opposée est ouverte pour la mise en place d'une rotule 3' solidaire d'une vis pédiculaire 4' implantée dans la vertèbre 6. Contre le fond de la chambre de compression 14 est fixé le bloc élastique 2 décrit précédemment de manière que sa face 2a solidaire de la coquille 2b coopère étroitement avec la rotule 3' de la vis 4' .

Le corps 13 est solidaire du côté de son extrémité ouverte d'une tige 15 en forme de U de manière que son extrémité arrondie 16 puisse venir en appui étroit contre une vis pédiculaire 4' ancrée dans l'autre vertèbre voisine 8 (fig. 4).

La vis pédiculaire 4' comporte au-dessus de sa partie filetée 4'a une butée circulaire 4'b qui délimite avec la rotule 3' un espace 4'c pour la retenue axiale de la partie recourbée 16 de la tige 15 (fig. 4).

L'extrémité ouverte du corps 13 peut être fermée par l'intermédiaire d'un bouchon ou d'une enveloppe ou d'un soufflet non représenté pour éviter toute pénétration de tissus vivants se trouvant à proximité de la chambre de compression 14.

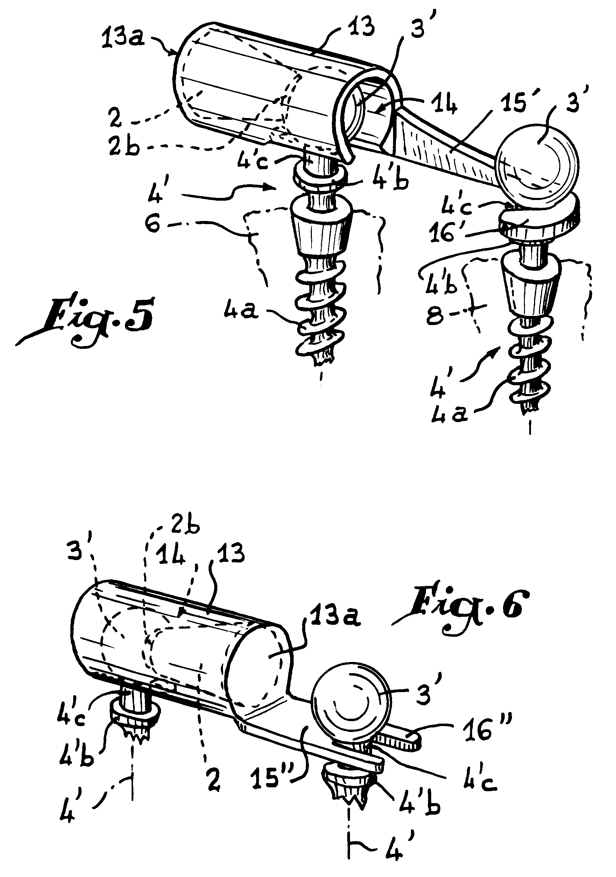

Une première variante de celle représentée en fig. 4 consiste en ce que le corps 13 soit solidaire du côté de son extrémité ouverte d'une tige horizontale 15' en forme de canne comportant une partie recourbée 16' dans un plan horizontal qui vient en appui contre une autre vis pédiculaire 4' implantée dans la vertèbre voisine 8 (fig. 5).

En fig. 6, on a illustré une seconde variante de la prothèse 13 représentée en fig. 4 qui consiste en ce que le fond 13a de la chambre 14 est solidaire sur sa face extérieure et dans le prolongement du corps 13 d'une tige 15'' à profil aplati dont l'extrémité libre 16'' présente la forme d'une fourche.

A l'intérieur du corps 13 et contre la face interne du fond 13a de la chambre 14 est fixé un bloc élastique 2 dont la face 2a solidaire de la coquille 2b est destinée à coopérer étroitement avec la rotule 3' de la vis pédiculaire 4' implantée dans la vertèbre 6. La fourche 16'' de la plaque 15'' est prévue pour coopérer avec l'espace 4'c de la vis pédiculaire 4' implantée dans la vertèbre 8 voisine de celle 6.

Le corps 13 représenté en fig. 6 fonctionne rigoureusement en sens inverses de ceux décrits préalablement. En effet, le bloc élastique 2 se comprime lorsque la distance séparant les deux rotules 3' des vis pédiculaires 4' voisines diminue.

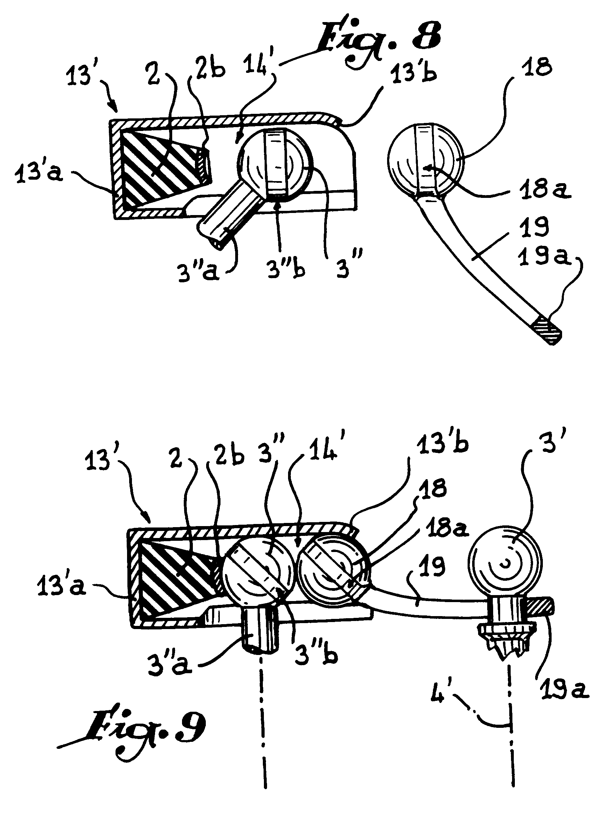

En fig. 7, on a montré une variante de fig. 4 qui consiste en ce qu'une tige 15''' est prévue indépendante du corps 13 pour être réglable en longueur par rapport à ce dernier. La tige 15''' coulisse à l'intérieur d'un guide 17 solidaire le long du corps 13. La tige 15''' est retenue du côté du fond 13a du corps 13 par un système d'arrêt adapté, comme par exemple un écrou.

La tige 15''' comporte une extrémité recourbée 16''' qui coopère avec l'espace libre 4'c d'une vis 4' .

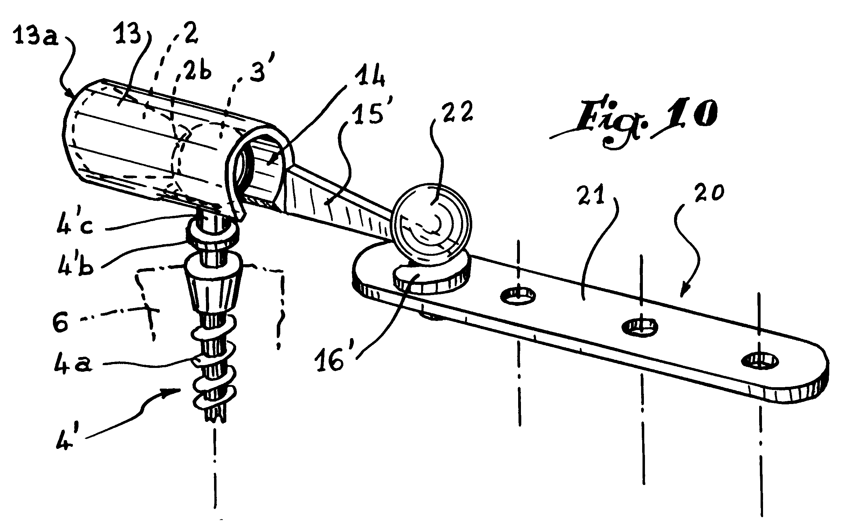

En fig. 8 et 9 , on a représenté un corps 13' comportant une chambre de compression 14' dont l'une des extrémités est fermée par un fond 13'a, tandis que celle opposé est ouverte et présente une déformation 13'b formant butée et réduisant la section d'entrée de la chambre 14' .

A l'intérieur du corps 13' est fixé contre son fond 13'a le bloc élastique 2, de manière que la coquille 2b coopère étroitement avec une rotule 3'' qui est solidaire d'une queue 3''a coopérant par exemple avec une vis pédiculaire 4 à l'aide d'une vis de pression 5, comme représenté en fig. 1.

La rotule 3'' présente un méplat équatorial 3''b permettant l'introduction de ladite rotule dans la chambre 14' . Le méplat 3''b oblige le chirurgien à introduire la rotule 3'' suivant la position montrée en fig. 8, puis à faire basculer ladite rotule 3'' autour de son axe pour que la queue 3''a se trouve dans une position verticale (fig. 9).

On comprend que dans cette position, la rotule 3'' soit retenue à l'intérieur de la chambre 14' du corps 13' .

Une seconde rotule 18 comprend un méplat équatorial 18a permettant son introduction dans la chambre 14' . De la même manière que pour la rotule 3'', la rotule 18 est introduite suivant la position montrée en fig. 8 et ensuite basculée suivant la position de fig. 9 pour être alors retenue à l'intérieur de la chambre 14' par la déformation 13'b du corps 13' . Le méplat 18a présente un diamètre identique à celui 3''b de la rotule 3''.

La rotule 18 est solidaire d'une plaque horizontale 19 ou tige qui présente à son extrémité libre 19a, c'est-à-dire à l'opposé de ladite rotule, soit une partie recourbée, soit une fourche ou un système d'amarrage approprié suivant l'utilisation du corps 13' .

L'extrémité libre de la plaque 19 est prévue pour coopérer avec l'espace 4'c d'une autre vis 4' implantée dans la vertèbre 8 voisine de celle 6.

Les différentes variantes décrites précédemment peuvent être associées entre elles pour constituer une prothèse intervertébrale extradiscale ayant la qualité d'amortir les déplacements entre les vertèbres 6 et 8 soit en compression, et en extension en permanence.

On note que les différents corps représentés en fig. 1 à 8 peuvent être combinés avec une tige ou une plaque 21 d'un fixateur d'ostéosynthèse 20 pour constituer un montage protecteur des extrémités d'un système rigide en évitant une trop grande concentration de contraintes sur certains étages. La tige ou plaque 21 comprend à l'une des extrémités libres une retenue 22 qui coopère avec les moyens de Liaison 9, 15, 15', 15'', 15''', 19 des différentes prothèses décrites préalablement (fig. 10). Dans notre exemple, la prothèse intervertébrale extradiscale représentée est celle de fig. 5. Bien évidemment il peut être prévu toutes les autres variantes sans pour cela changer la combinaison.

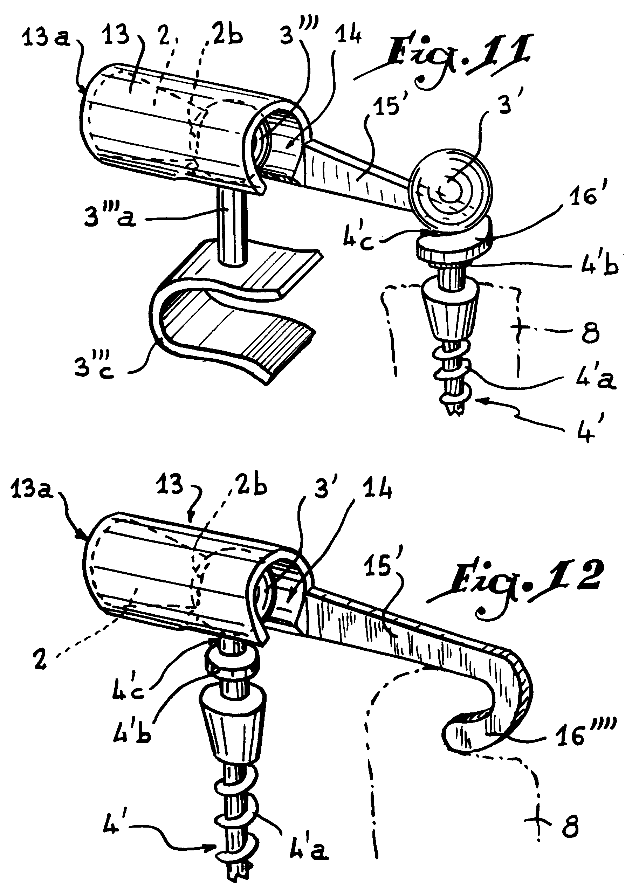

En fig. 11, on a représenté une variante des moyens de fixation dans les vertèbres 6 et 8. En effet, la rotule 3''' par l'intermédiaire de sa queue 3'''a peut être solidaire d'un crochet 3'''c qui vient s'accrocher directement sur les lames des vertèbres à réunir, réalisant ainsi un dispositif d'ancrage sous-lamaire.

En fig. 12, on a montré une variante de celle de fig. 5 dont l'extrémité de la tige 15' est recourbée en forme de crochet 16'''' dans un plan vertical pour venir directement s'accrocher sous une lame osseuse de la vertèbre voisine 8 à réunir.

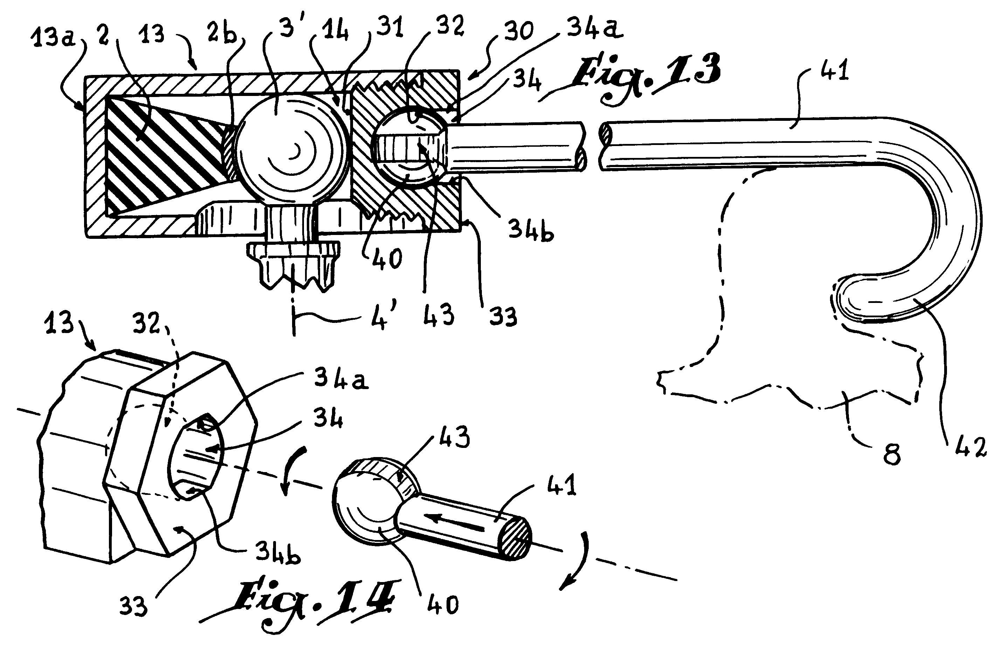

On a illustré en fig. 13 et 14 une variante de celle représentée en fig. 8 et 9. La prothèse extradiscale comprend un corps rigide 13 à profil cylindrique dont le fond 13a de la chambre 14 reçoit le bloc élastique 2. Ce dernier est solidaire d'une coquille ou analogue 2b qui est destinée à coopérer étroitement avec la rotule 3' associée à une vis pédiculaire 4' . L'extrémité opposée au fond 13a du corps 13 est fermée par l'intermédiaire d'un bouchon fileté 30 qui permet de rendre étanche la chambre 14 lorsque la vis 4' est associée à un soufflet d'étanchéité non représenté. Le bouchon 30 présente une face arrière 31 qui vient en appui contre la rotule 3' de manière à maintenir axialement cette dernière à l'intérieur de la chambre 14. A l'intérieur du bouchon 30 est percé un logement sphérique 32 qui débouche du côté de la face avant 33 opposée à celle arrière 31 par l'intermédiaire d'une ouverture 34.

L'ouverture 34 présente un profil cylindrique pourvu de deux méplats opposés et parallèles 34a et 34b distants l'un de l'autre d'une cote inférieure au diamètre du logement sphérique 32 (fig. 14).

Le diamètre de l'ouverture cylindrique 34 est égal à ceux du logement sphérique 32 et de la rotule 40.

A l'intérieur du logement 32 est retenue une rotule 40 solidaire d'une tige 41 dont l'extrémité libre 42 présente la forme d'un crochet pour venir coopérer directement sous la lame osseuse d'une vertèbre voisine 8. Bien évidemment, l'extrémité libre 42 peut affecter d'autres formes comme par exemple une fourche ou un crochet pour venir coopérer avec une vis pédiculaire non représentée.

La rotule 40 comporte un méplat équatorial 43 dont le diamètre est sensiblement équivalent à la distance séparant les méplats 34a et 34b de l'ouverture 34. On constate que le méplat 43 de la rotule 40 permettant l'introduction de celle-ci à l'intérieur du logement sphérique 32 est usiné dans un plan perpendiculaire à celui d'accrochage de l'extrémité libre 42 (fig. 13).

En effet, le chirurgien oriente la rotule 40 de manière que le méplat 43 corresponde avec ceux 34a et 34b pour sa mise en place à l'intérieur du bouchon 30. Cette position permet l'introduction de la rotule, tandis que pour sa retenue, il suffit de faire pivoter la rotule 40 d'un quart de tour pour que sa partie sphérique vienne en butée contre les méplats 34a et 34b distants d'une cote inférieure au diamètre de la rotule 40 (fig. 13).

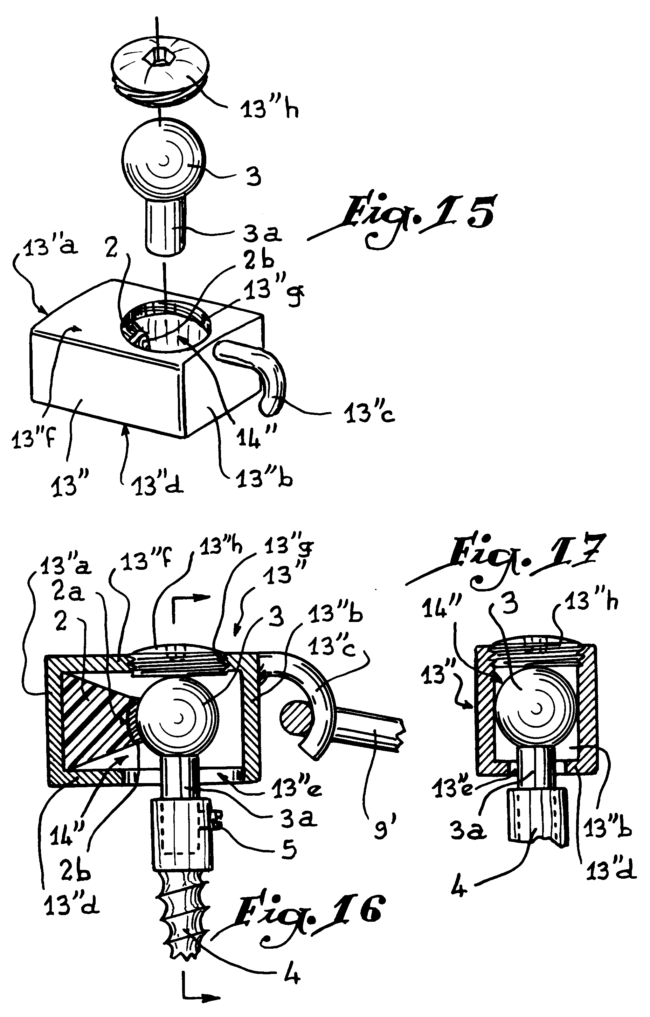

On a représenté en fig. 15 à 17 une dernière variante de la prothèse intervertébrale extradiscale qui comprend un corps rigide 13'' dont le fond 13''a de la chambre 14'' reçoit le bloc élastique 2. L'extrémité opposée au fond 13''a du corps 13'' est fermée par l'intermédiaire d'une paroi 13''b dont la face extérieure est solidaire d'une anse 13''c qui peut être ouverte ou fermée. Cette dernière peut-être disposée dans un plan soit vertical soit horizontal. La face inférieure 13''d du corps 13'' reliant le fond 13''a et la paroi 13''b est percée d'une fente 13''e débouchant à l'intérieur de la chambre 14''. A l'opposé de la fente 13''e, la face supérieure 13''f du corps 13'' est percée d'un trou fileté 13''g pour le passage par exemple de la rotule 3 solidaire de la tige 3a qui vient se porter dans une vis 4 préalablement fixée dans une vertèbre non représentée.

Le trou fileté 13''g est d'un diamètre supérieure à celui de la rotule 3 pour permettre sa mise en place à l'intérieur de la chambre 14''. La rotule 3' est disposée dans la chambre 14'' pour venir en appui contre la coquille 2b du bloc élastique 2, tandis que la tige 3a coopère avec la fente 13''e.

Le trou fileté 13''g reçoit un bouchon fileté 13''h pour fermer la chambre 14'' lorsque la rotule 3 est introduite. La tige 3a peut recevoir un manchon non représenté pour rendre la chambre 14'' totalement étanche.

L'anse 13''c est prévue pour coopérer avec un maillon 9 de forme quelconque reliant le corps 13'' à une autre vis 4 ou 4' solidaire d'une autre vertèbre.

On constate que les tiges 15, 15', 15'', 15''' et 41 et la plaque 19 présentent une forme soit de crochet, soit de fourche qui sont susceptibles de venir en appui contre une vis pédiculaire 4, 4' ou de s'ancrer directement dans l'os ou sous les lames des vertébres à réunir.

On note que le bloc élastique 2 peut prendre n'importe quelle forme, sans changer ses caractéristiques. De plus, le bloc élastique 2 peut être associé avec un ressort non représenté pour améliorer ses caractéristiques élastiques. En outre, la matière du bloc 2 peut varier suivant les conditions élastiques recherchées et être combinée avec par exemple des fibres composites.

Enfin, au cas où le disque intervertébral 11 situé entre les vertèbres 6 et 8 serait partiellement effondré, on pourrait lui adjoindre une cale élastique 12 de forme quelconque rétablissant un écartement plus normal entre les vertèbres et qui agirait en combinaison avec la prothèse intervertébrale extradiscale sous quelque forme que ce soit pour soulager le rachis (fig. 3).

## Revendications

1. Prothèse intervertébrale extradiscale munie de moyens d'accrochage directs ou indirects à deux vertèbres du rachis d'un patient, comprenant un corps allongé (1, 1', 13, 13', 13'') totalement ou partiellement fermé comportant une chambre de compression (1e, 1'e, 14, 14', 14''), et une rotule (3, 3'' et 3', 3''') associée à un premier moyen de fixation (4 et 41, 3'''c) engagé dans une première vertèbre (6), ledit corps l'étant accroché à une autre vertèbre (8) ou étant relié à un autre moyen de fixation (4 et 4', 3'''c) implanté dans une autre vertèbre (8), directement ou indirectement par l'intermédiaire d'au moins un moyen de liaison (9, 15, 15', 15'', 15''', 19 et 41) qui est en appui contre ledit autre moyen de fixation (4 et 4', 3'''c), caractérisée en ce que ladite rotule (3, 3'' et 3', 3''') est retenue à l'intérieur de la chambre de compression (1e, 1'e, 14, 14', 14''), une unique extrémité (1b, 13a, 13''a) de ladite chambre étant solidaire d'un bloc élastique (2) dont une face (2a) est en appui contre la rotule.

2. Prothèse suivant la revendication 1, caractérisée en ce que la face libre (2a) du bloc élastique (2) affecte la forme d'une portion concave de sphère de même diamètre que celui de la rotule (3, 3', 3'', 3''').

3. Prothèse suivant la revendication 2, caractérisée en ce que la face libre (2a) du bloc élastique (2) est pourvue d'une coquille rigide (2b) ayant la forme d'une calotte sphérique concave de diamètre égal à celui de la rotule (3, 3', 3'', 3''').

4. Prothèse suivant la revendication 1, caractérisée en ce que l'extrémité (1c) du corps (1) opposée à celle qui porte le bloc élastique (2) est destinée à prendre appui contre une tige (7) en forme de crochet solidaire de la vis pédiculaire (4) implantée dans l'autre vertèbre (8).

5. Prothèse suivant la revendication 4, caractérisée en ce que la tige (7) comporte une tête (7a) s'étendant au-dessus de la partie extrême correspondante (1c) du corps (1) et se trouvant en appui sur sa face supérieure.

6. Prothèse suivant la revendication 1, caractérisée en ce que l'extrémité (1'c) du corps (1') opposée à celle (1'a) qui porte le bloc élastique (2) est reliée à la vis pédiculaire (4) solidaire de l'autre vertèbre (8) au moyen d'un maillon (9).

7. Prothèse suivant la revendication 1, caractérisée en ce qu'elle comporte un corps cylindrique (13) solidaire dans son prolongement, soit du côté de son extrémité ouverte, soit du côté de son fond (13a), d'une tige (15, 15', 15'', 15''') comprenant une extrémité (16, 16', 16'', 16''') qui vient en appui contre une autre vis pédiculaire (4') implantée dans la vertèbre voisine (8).

8. Prothèse suivant la revendication 7, caractérisée en ce que la tige (15) présente la forme d'un U de manière que son extrémité arrondie (16) puisse venir en appui étroit contre une vis pédiculaire (4').

9. Prothèse suivant la revendication 7, caractérisée en ce que la tige (15') présente la forme d'une canne dont l'extrémité recourbée (16') vient en appui contre une vis pédiculaire (4').

10. Prothèse suivant la revendication 7, caractérisée en ce que la tige (15'') présente un profil aplati dont l'extrémité libre (16'') est conformée suivant une fourche.

11. Prothèse suivant la revendication 7, caractérisée en ce que la tige (15''') présente une extrémité libre recourbée (16''').

12. Prothèse suivant la revendication 7, caractérisée en ce que le corps (13) est solidaire d'un guide (17) pour le coulissement de la tige (15'''), ladite tige étant retenue par un système adapté sur le corps (13).

13. Prothèse suivant la revendication 1, caractérisée en ce qu'elle comprend un corps cylindrique (13') dont la chambre de compression (14') est prévue pour recevoir une seconde rotule (18) qui vient en appui contre la première (3''), ladite seconde rotule (18) étant solidaire d'une plaque (19) qui présente à son extrémité libre soit une partie recourbée, soit une fourche (19a).

14. Prothèse suivant la revendication 13, caractérisée en ce que le corps (13') comporte du côté de son extrémité ouverte (13'b) une réduction de diamètre formant butée.

15. Prothèse suivant la revendication 13, caractérisée en ce que les rotules (3'' et 18) comportent respectivement un méplat équatorial (3'b et 18a) et dont le diamètre est sensiblement équivalent à celui de l'extrémité ouverte du corps (13'b) pour leur introduction à l'intérieur de la chambre de compression (14').

16. Prothèse suivant la revendication 1, caractérisée par sa combinaison avec une cale élastique (12) engagée au niveau du disque intervertébral (11) entre les deux vertèbres (6, 8) qui reçoivent les vis pédiculaires (4, 4').

17. Prothèse suivant la revendication 1, caractérisée en ce que le moyen de liaison (9, 15, 15', 15'', 15''', 19) reliant le corps à l'autre vertèbre, coopère avec une tige ou une plaque (21) d'un fixateur d'ostéosynthèse (20).

18. Prothèse suivant la revendication 1, caractérisée en ce que l'extrémité ouverte du corps (1, 1', 13, 13') peut être fermée par l'intermédiaire d'un bouchon, d'une enveloppe ou d'un soufflet pour éviter toute pénétration de tissus vivants se trouvant à proximité de la chambre de compression (1e, 1'e, 14, 14').

19. Prothèse suivant la revendication 1, caractérisée en ce que les moyens de fixation sont constitués d'une vis pédiculaire (4) dans lesquel les sont introduites des tiges (3a, 3''a, et 7) comprenant respectivement une rotule (3, 3'') et une tête (7a).

20. Prothèse suivant la revendication 1, caractérisée en ce que les moyens de fixation sont constitués de vis pédiculaires (4') comprenant au-dessus de la partie filetée (4'a) une butée circulaire (4'b) qui délimite avec la rotule (3') un espace (4'c).

21. Prothèse suivant la revendication 1, caractérisée en ce que les moyens de fixation sont constitués d'une rotule (3'') solidaire d'un crochet (3'''c) par l'intermédiaire de sa queue (3'''a) pour sa mise en place directement sur la vertèbre à réunir.

22. Prothèse suivant la revendication 1, caractérisée en ce que la tige (15') comprend une extrémité recourbée (16'''') dans un plan vertical pour venir s'accrocher directement sous une lame osseuse de la vertèbre à réunir.

23. Prothèse suivant la revendication 1, caractérisée en ce que le corps (13) est solidaire du côté opposé à son fond (13a) d'un bouchon (30) comprenant des moyens pour la retenue d'une tige (41).

24. Prothèse suivant la revendication 23, caractérisée en ce que le bouchon (30) comporte une face (13) qui vient en appui contre la rotule (3') préalablement placée dans le corps (13), tandis que sa face opposée (33) est percée d'un logement sphérique (32) pourvu d'une ouverture cylindrique (34) comprenant deux méplats opposés et parallèles (34a, 34b).

25. Prothèse suivant la revendication 24, caractérisée en ce que le logement (32) est prévu pour recevoir une rotule (40) solidaire de la tige (41), ladite rotule comprenant un méplat équatorial (43) pour permettre son introduction dans ledit logement.

26. Prothèse suivant la revendication 25, caractérisée en ce que le méplat (43) de la rotule (40) est usiné suivant un plan perpendiculaire à celui d'accrochage (42) de la tige (41).

27. Prothèse suivant la revendication 1, caractérisée en ce que le bloc élastique (2) est associé à un ressort.

28. Prothèse suivant la revendication 1, caractérisée en ce qu'elle comporte un corps (13'') fermé à chaque extrémitée par un fond (13''a) et une parois (13''b) dont la face extérieure est solidaire d'une anse (13''c), tandis que la face inférieure (13''d) est percée d'une fente (13''e) et la face supérieure (13''f) est pourvue d'un trou fileté (13''g) pour la mise en place de la rotule (3) à l'intérieur de la chambre (14'').

29. Prothèse suivant la revendication 28, caractérisée en ce que le trou fileté (13''g) reçoit un bouchon fileté (13''h) pour fermer la chambre (14'').

30. Prothèse suivant la revendication 1, caractérisée en ce que la chambre de compression (1e, 1'e) est pourvue d'une empreinte (1f, 1'f) pour la retenue de la rotule (3).

31. Prothèse suivant la revendication 4, caractérisée en ce que l'extrémité (1c) est pourvue de deux empreintes opposées (1g, 1h) pour la retenur d'une rotule (7'a) solidaire d'une tige (7') qui vient se fixer dans la vis pédiculaire (4).

32. Prothèse suivant la revendication 1, caractérisée en ce que la rotule (3) est fixée sur une queue (3'a) à profil conique par l'intermédiaire d'un écrou (3'c).

## Claims

1. An intervertebral extradiscal prosthesis provided with means for engagement directly or directly to two spinal vertebrae of a patient, comprising a completely or partially closed elongate body (1, 1', 13, 13', 13'') comprising a compression chamber (1e, 1'e, 14, 14', 14''), and a ball head (3, 3'' and 3', 3''') associated with a first fixing means (4, 4' and 3'''c) engaged in a first vertebra (6), the said body being attached to another vertebra (8) or being connected with another fixing means (4 and 4', 3''c) implanted in another vertebra (8), directly or indirectly by the intermediary of at least one connecting means (9, 15, 15', 15'', 15''', 19 and 41) which is in engagement with the said other fixing means (4 and 4', 3'''c), characterised in that the said ball head (3, 3'' and 3', 3''') is retained inside the compression chamber (1e, 1'e, 14, 14', 14''), one single end (1b, 13a, 13''a) of the said chamber being fixed to an elastic block (2) of which one face (2a) is in engagement with the ball head.

2. Prosthesis according to claim 1, characterised in that the free face (2a) of the elastic block (2) has the form of a concave portion of a sphere of the same diameter as that of the ball head (3, 3', 3'', 3''').

3. Prosthesis according to claim 2, characterised in that the free face (2a) of the elastic block (2) is provided with a rigid shell (2b) having the form of a concave spherical cap of diameter equal to that of the spherical head (3, 3', 3'', 3''').

4. Prosthesis according to claim 1, characterised in that the end (1c) of the body (1) opposite to that which carries the elastic block (2) is intended to come into engagement with a stem (7) in the form of a hook fixed to the pedicle screw (4) implanted in the other vertebra (8).

5. Prosthesis according to claim 4, characterised in that the stem (7) comprises a head (7a) disposed over the corresponding extreme part (1c) of the body (1) and positioned in engagement with the upper face thereof.

6. Prosthesis according to claim 1, characterised in that the end (1'c) of the body (1') opposite to that (1'a) which carries the elastic block is connected by means of a link (9) to the pedicle screw (4) fixed to the other vertebra.

7. Prosthesis according to claim 1, characterised in that it comprises a cylindrical body (13) fixedly extended, either from its open end or from its base (13), by a stem (15, 15', 15'', 15''') comprising an extremity (16, 16', 16'', 16''') which comes into engagement with another pedicle screw (4') implanted in a neighbouring vertebra.

8. Prosthesis according to claim 7, characterised in that the stem (15) has the form of a U such that its curved end (16) can come into tight engagement with a pedicle screw (4').

9. Prosthesis according to claim 7, characterised in that the stem (15') has the form of a cane of which the curved end (16) can come into engagement with a pedicle screw (4').

10. Prosthesis according to claim 7, characterised in that the stem (15'') has a flat profile of which the free end (16'') is shaped like a fork.

11. Prosthesis according to claim 7, characterised in that the stem (15''') has a curved free end (16''').

12. Prosthesis according to claim 7, characterised in that the body (13) is fixed to a guide (17) through which the stem (15''') runs, the said stem being retained by a system adapted to the body (13).

13. Prosthesis according to claim 1, characterised in that it comprises a cylindrical body (13') of which the compression chamber (14') is adapted to receive a second ball head (18) which comes into engagement with the first (3''), the said second ball head (18) being fixed to a plate (19) which has at its free end either a curved part or a fork (19a).

14. Prosthesis according to claim 13, characterised in that the body comprises on the side of its open extremity (13'b) a reduction in diameter forming an abutment.

15. Prosthesis according to claim 13, characterised in that the ball heads (13'' and 18) comprise a respective planar equatorial zone (3'b and 18a) of which the diameter is substantially equivalent to that of the open end of the body (13'b) for its introduction within the compression chamber (14').

16. Prosthesis according to claim 1, characterised by the combination with an elastic wedge (12) engaged at the level of an intervertebral disc (11) between the two vertebrae (6, 8) which receive the pedicle screws (4, 4').

17. Prosthesis according to claim 1, characterised in that the connecting means (9, 15, 15', 15'', 15''', 19) connecting the body to the other vertebra cooperates with a stem or a plate (21) of an osteosynthetic setter (20).

18. Prosthesis according to claim 1, characterised in that the open end of the body (1, 1', 13, 13') can be closed by means of a stopper, an envelope or a gusset to avoid any penetration of living tissue which is in the region of the compression chamber (1e, 1'e, 14, 14').

19. Prosthesis according to claim 1, characterised in that the fixing means are constituted by a pedicle screw (4) into which are introduced stems (3a, 3''a and 7) comprising respectively a ball head (3, 3") and a head (7a).

20. Prosthesis according to claim 1, characterised in that the fixing means are constituted by pedicle screws (4') comprising above the threaded part (4'a) a circular abutment (4'b) which delimits a space (4'c) with the ball head (3').

21. Prosthesis according to claim 1, characterised in that the fixing means are constituted by a ball head (3'') fixed to a hook (3'''c) by means of its stem (3'''a) for its direct disposition on the vertebra to be joined.

22. Prosthesis according to claim 1, characterised in that the stem (15') comprises a hooked end (16'''') in a vertical plane to come into direct engagement with a bony blade of the vertebra to be joined.

23. Prosthesis according to claim 1, characterised in that the body (13) is fixed at the side opposite its base (13a) with a stopper (30) comprising means for retention of a stem (41).

24. Prosthesis according to claim 23, characterised in that the stopper (30) comprises a face (31) which comes into engagement with the ball head (3') previously placed in the body (13), whereas the opposite face (33) is pierced by a spherical socket (32) provided with a cylindrical opening (34) comprising two parallel and opposite planar zones (34a, 34b).

25. Prosthesis according to claim 24, characterised in that the socket (32) is adapted to receive a spherical head (40) fixed to the stem (41), the said ball head comprising an planar equatorial zone (43) for enabling its introduction into the said socket.

26. Prosthesis according to claim 25, characterised in that the planar zone (43) of the ball head (40) is disposed in a plane perpendicular to that of the hook (42) of the stem (41).

27. Prosthesis according to claim 1, characterised in that the elastic block (2) is associated with a spring.

28. Prosthesis according to claim 1, characterised in that it comprises a body (13'') closed at each end by a base (13''a) and an enclosure (13''b) of which the outer face is fixed to a handle (13''c), whereas the lower face (13''d) is pierced by a slot (13''e) and the upper face (13''f) is provided with a screw-threaded hole (13''g) for the insertion of the baldhead (3) within the chamber (14'').

29. Prosthesis according to claim 28, characterised in that the threaded hole (13''g) receives a threaded stopper (13''h) for closure of the chamber (14).

30. Prosthesis according to claim 1, characterised in that the compression chamber (1e, 1'e) is provided with a track (1f, 1'f) for the retention of the ball head (3).

31. Prosthesis according to claim 4, characterised in that the end (1c) is provided with two opposed tracks (1g, 1h) for the retention of a ball head (7'a) fixed to a stem (7') which can be fixed in the pedicle screw (4).

32. Prosthesis according to claim 1, characterised in that the ball head (3) is fixed on a conically shaped stem (3'a) by means of a nut (3'c).

## Patentansprüche

1. Intervertebrale, außerhalb der Bandscheibe angeordnete Prothese, ausgerüstet mit Befestigungsmitteln direkt oder indirekt an zwei Wirbeln der Wirbelsäule eines Patienten, mit einem langgestreckten, teilweise oder insgesamt abgeschlossenen Körper (1,1',13,13',13''), der eine Kompressionskammer (1e,1'e,14,14',14'') umfaßt, und einer Kupplungskugel (3,3'' und 3',3'''), die einem ersten mit einem ersten Wirbel (6) verbundenen Befestigungsmittel (4 und 4',3'''c) zugeordnet ist, wobei der Körper an einem anderen Wirbel (8) befestigt oder mit einem anderen Befestigungsmittel (4 und 4',3''') verbunden ist, das direkt oder indirekt über mindestens ein sich gegen das andere Befestigungsmittel (4 und 4',3''') abstützendes Verbindungsmittel (9,15,15',15'',15''', 19 und 41) in einen anderen Wirbel (8) eingesetzt ist,
**dadurch gekennzeichnet,**
daß die Kupplungskugel (3,3'' und 3',3''') im Inneren der Kompressionskammer (1e,1'e,14,14',14'') gehalten ist, wobei ein einziges Ende (1b,13a,13''a) der Kammer fest mit einem elastischen Block (2) verbunden ist, dessen eine Fläche (2a) sich gegen die Kupplungskugel abstützt.

2. Prothese nach Anspruch 1, dadurch gekennzeichnet, daß die freie Fläche (2a) des elastischen Blocks (2) die Form eines konkaven Kugelteils gleichen Durchmessers wie der der Kupplungskugel (3,3',3'',3''') aufweist.

3. Prothese nach Anspruch 2, dadurch gekennzeichnet, daß die freie Fläche (2a) des elastischen Blocks (2) mit einer starren Schale (2b) versehen ist, die die Form einer konkaven Kugelkalotte mit einem dem Durchmesser der Kupplungskugel (3,3',3'',31''') entsprechenden Durchmesser aufweist.

4. Prothese nach Anspruch 1, dadurch gekennzeichnet, daß das dem den elastischen Block (2) tragenden Ende gegenüberliegende Ende (1c) des Körpers (1) in Form eines Hakens abstützt, der mit der in den anderen Wirbel (8) eingesetzten Stielschraube (4) verbunden ist.

5. Prothese nach Anspruch 4, dadurch gekennzeichnet, daß der Stiel (7) einen Kopf (7a) aufweist, der sich über das entsprechende Endteil (1c) des Körpers (1) erstreckt und sich an seiner oberen Fläche abstützt.

6. Prothese nach Anspruch 1, dadurch gekennzeichnet, daß das Ende (1'c) des Körpers (1'), das dem den elastischen Block (2) tragenden Ende (1'a) gegenüberliegt, mit einer Stielschraube (4) verbunden ist, die mittels einer Schake (9) an dem anderen Wirbel (8) befestigt ist.

7. Prothese nach Anspruch 1, dadurch gekennzeichnet, daß sie einen zylindrischen Körper (13) umfaßt, der in seiner Verlängerung, sei es an der Seite seines offenen Endes, sei es an der Seite seines Bodens (13a), mit einem Stiel (15,15',15'',15''') verbunden ist, der ein sich gegen eine andere Stielschraube (4') abstützendes Ende (16,16',16'',16''') umfaßt, wobei die Stielschraube in dem benachbarten Wirbel (8) eingesetzt ist.

8. Prothese nach Anspruch 7, dadurch gekennzeichnet, daß der Stiel (15) eine U-Form aufweistderart, daß sein abgerundetes Ende (16) sich eng gegen eine Stielschraube (4') abstützen kann.

9. Prothese nach Anspruch 17, dadurch gekennzeichnet, daß der Stiel (15') die Form eines Krückstockes aufweist, dessen abgerundetes Ende (16') sich gegen eine Stielschrauben (4') abstützt.

10. Prothese nach Anspruch 7, dadurch gekennzeichnet, daß der Stiel (15'') ein abgeflachtes Profil aufweist, dessen freies Ende (16'') entsprechend einer Gabel geformt ist.

11. Prothese nach Anspruch 7, dadurch gekennzeichnet, daß der Stiel (15''') ein freies gekrümmtes Ende (16''') aufweist.

12. Prothese nach Anspruch 7, dadurch gekennzeichnet, daß der Körper (13) mit einer Führung (17) für die gleitende Verschiebung des Stiels (15''') verbunden ist, wobei der Stiel durch ein angepaßtes System auf dem Körper (13) zurückgehalten wird.

13. Prothese nach Anspruch 1, dadurch gekennzeichnet, daß sie einen zylindrischen Körper (13') umfaßt, dessen Druckkammer (14') ausgebildet ist, um eine zweite Kupplungskugel (18) aufzunehmen, die sich gegen die erste (3''') abstützt, wobei die zweite Kupplungskugel (18) mit einer Platte (19) verbunden ist, die an ihrem freien Ende ein gekrümmtes Teil oder eine Gabel (19a) aufweist.

14. Prothese nach Anspruch 13, dadurch gekennzeichnet, daß der Körper (13') an der Seite seines offenen Endes (13'b) eine Durchmesserverringerung aufweist, die einen Anschlag bildet.

15. Prothese nach Anspruch 13, dadurch gekennzeichnet, daß die Kupplungskugeln (3'' und 18) jeweils eine äquatoriale Abflachung (3'b, und 18a) umfassen, deren Durchmesser für ihre Einführung ins Innere der Kompressionskammer (14') im wesentlichen gleich dem des offenen Endes des Körpers (13'b) ist.

16. Prothese nach Anspruch 1, gekennzeichnet durch ihre Kombination mit einem elastischen Klotz (12), der an der Bandscheibe zwischen die zwei Wirbel (6,8), die die Stielschrauben (4,4') aufnehmen, eingesetzt ist.

17. Prothese nach Anspruch 1, dadurch gekennzeichnet, daß das Verbindungsmittel (9,15,15',15'',15''',19), das den Körper mit dem anderen Wirbel verbindet, mit einem Stiel oder eine Platte (21) eines osteosynthetischen Fixateurs zusammenarbeitet.

18. Prothese nach Anspruch 1, dadurch gekennzeichnet, daß das offene Ende des Körpers (1,1',13,13') mittels eines Stopfens, einer Umhüllung oder eines Balges geschlossen werden kann, um jedes Eindringen von lebendem Gewebe, das sich in der Nähe der Kompressionskammer (1e,1'e,14,14') befindet, zu vermeiden.

19. Prothese nach Anspruch 1, dadurch gekennzeichnet, daß die Befestigungsmittel aus einer Stielschraube (4) gebildet werden, in die Stiele (3a,3'' und 7) eingeführt sind, die jeweils eine Kupplungskugel (3,3'') und einen Kopf (7a) umfassen.

20. Prothese nach Anspruch 1, dadurch gekennzeichnet, daß die Befestigungsmittel aus Stielschrauben (4') gebildet werden, die über den Gewindeteil (4'a) einen kreisförmigen Anschlag (4'b) umfassen, der mit der Kupplungskugel (3') einen Raum (4'c) begrenzt.

21. Prothese nach Anspruch 1, dadurch gekennzeichnet, daß die Befestigungsmittel von einer Kupplungskugel (3''') gebildet werden, die für ihre Instellungbringung direkt auf dem zu verbindenden Wirbel unter Zwischenschaltung ihres Verankerungsendes (3'''a) mit einem Haken (3'''c) fest verbunden ist.

22. Prothese nach Anspruch 1, dadurch gekennzeichnet, daß der Stiel (15') in einer vertikalen Ebene ein gekrümmtes Ende (16'''') umfaßt, um sich direkt unter einem Knochenvorsprung des zu verbindenden Wirbels festzuhaken.

23. Prothese nach Anspruch 1, dadurch gekennzeichnet, daß der Körper (13) gegenüber seinem Boden (13a) mit einem Stopfen (30) verbunden ist, der Mittel zum Halten eines Stiels (41) umfaßt.

24. Prothese nach Anspruch 23, dadurch gekennzeichnet, daß der Stopfen (30) eine Fläche (31) umfaßt, die sich gegen die Kupplungskugel (3') abstützt, die vorher im Körper (13) angeordnet wurde, während seine gegenüberliegende Fläche (33) von einer sphärischen Aufnahme (32) durchdrungen ist, die mit einer zylindrischen Öffnung (34) versehen ist, die zwei gegenüberliegende und parallele Abflachungen (34a,34b) aufweist.

25. Prothese nach Anspruch 24, dadurch gekennzeichnet, daß die Aufnahme (32) vorgesehen ist, um eine mit dem Stiel (41) fest verbundene Kupplungskugel (40) aufzunehmen, wobei die Kupplungskugel eine äquatoriale Abflachung aufweist, die eine Einführung in die Aufnahme erlaubt.

26. Prothese nach Anspruch 25, dadurch gekennzeichnet, daß die Abflachung (43) der Kupplungskugel (40) entsprechend einer senkrechten Ebene zu der der Befestigung (42) des Stiels (41) bearbeitet ist.

27. Prothese nach Anspruch 1, dadurch gekennzeichnet, daß der elastische Block (2) einer Feder zugeordnet ist.

28. Prothese nach Anspruch 1, dadurch gekennzeichnet, daß sie einen Körper (13'') umfaßt, der an jedem Ende durch einen Boden (13''a) und eine Wand (13''b) abgeschlossen ist, deren Außenfläche mit einem Bügel (13''c) verbunden ist, während die untere Fläche (13''d) von einem Schlitz (13''e) durchdrungen ist und die obere Fläche (13''f) mit einem Gewindeloch (13''g) für die Instellungbringung der Kupplungskugel im Inneren der Kammer (14'') versehen ist.

29. Prothese nach Anspruch 28, dadurch gekennzeichnet, daß das Gewindeloch (13''g) einen mit Gewinde versehenen Stopfen (13''h) aufnimmt, um die Kammer (14'') zu verschließen.

30. Prothese nach Anspruch 1, dadurch gekennzeichnet, daß die Kompressionskammer (1e,1'e) mit einem Eindruck (1f,1'f) für das Halten der Kupplungskugel (3) versehen ist.

31. Prothese nach Anspruch 4, dadurch gekennzeichnet, daß das Ende (1c) mit zwei gegenüberliegenden Eindrücken (1g,1h) zur Aufnahme einer mit einem Stiel (7') fest verbundenen Kupplungskugel (7'a) versehen ist, der in der Stielschraube (4) fixiert wird.

32. Prothese nach Anspruch 1, dadurch gekennzeichnet, daß die Kupplungskugel (3) auf einem Befestigungsansatz (3'a) mit konischem Profil mittels einer Schraube (3'c) befestigt ist.
